# EUROPEAN PATENT SPECIFICATION

(11) **EP 1 748 774 B1**
(45) Date of publication and mention of the grant of the patent: **15.12.2010**
(21) Application number: 05743969.7
(22) Date of filing: 24.05.2005
(51) Int. Cl.: A61K 31/353, A61K 31/352, A61P 9/10, A61P 3/06, A61K 31/355

(54) **Compositions comprising flavonoids and tocotrienols and uses thereof**
Flavonoide und Tocotrienole enthaltende Zusammensetzungen und deren Verwendungen
Compositions comprenant des flavonoides et des tocotrienols et utilisations associees

(30) Priority: 26.05.2004 US 574490 P
(43) Date of publication of application: 07.02.2007
(73) Proprietor: KGK Synergize, Inc., London, ON N6A 5R8 (CA)
(72) Inventor: GUTHRIE, Najla, London, Ontario N6B 3L5 (CA)
(74) Representative: Hüttermann, Aloys
(86) International application number: PCT/IB2005/001427
(87) International publication number: WO 2005/115378

(56) References cited:
- EP-A- 1 415 549
- WO-A-00/72862
- WO-A-01/32160
- WO-A-01/70029
- WO-A-02/34072
- WO-A-99/15167
- WO-A-02/055071
- WO-A1-02/055071
- WO-A2-02/34072
- CA-A1- 2 403 548
- US-A- 4 603 142
- US-A- 5 217 992
- US-A1- 2004 176 311
- MONFORTE M T ET AL: "BIOLOGICAL EFFECTS OF HESPERIDIN, A CITRUS FLAVONOID, (NOTE II): HYPOLIPIDEMIC ACTIVITY ON EXPERIMENTAL HYPERCHOLESTEROLEMIA IN RAT" IL FARMACO, ROME, IT, vol. 50, no. 9, September 1995 (1995-09), pages 595-599, XP002066945 ISSN: 0014-827X
- BORRADAILE N ET AL: "REGULATORY EFFECTS OF CITRUS FLAVONOIDS ON APO B METABOLISM IN HEPG2 CELLS" 17 March 1998 (1998-03-17), FASEB JOURNAL, FED. OF AMERICAN SOC. FOR EXPERIMENTAL BIOLOGY, BETHESDA, MD, US, PAGE(S) A207 , XP000999743 ISSN: 0892-6638 * the whole document *
- KUROWSKA E M ET AL: "ROLE OF TOCOTRIENOLS FROM PALM OIL IN REGULATION OF APO B METABOLISM IN HEPG2 CELLS" 12 March 1999 (1999-03-12), FASEB JOURNAL, FED. OF AMERICAN SOC. FOR EXPERIMENTAL BIOLOGY, BETHESDA, MD, US, PAGE(S) A562 , XP000999072 ISSN: 0892-6638 * the whole document *
- KUROWSKA E. ET AL: 'Hypolipidemic Effects and Absorption of Citrus Polymethoxylated Flavones in Hamsters with Diet-Induced Hypercholesterolemia' J. AGR. FOOD CHEM. vol. 52, 2004, pages 2879 - 2886, XP003015254
- KERCKHOFFS D. ET AL: 'Effects on the Human Serum Lipoprotein Profile of beta-Glucan, Soy Protein and Isoflavones, Plant Sterols and Stanols, Garlic and Tocotrienols' J. NUTR. vol. 132, no. 9, September 2002, pages 2494 - 2505, XP002459647

## Description

### BACKGROUND OF THE INVENTION

Hyperlipidemia is a pathological state in mammals, where there is an abnormally high concentration of lipids circulating in the serum. The composition of the lipid pool in the circulation consists mostly of triglyceride (fatty acid esters of glycerol), cholesterol, and fatty acid esters of cholesterol. Such molecules are generally found bound to specific proteins in the form of complexes which act as transporting mechanisms. Hyperlipidemia is a condition which is commonly associated with elevated levels of cholesterol, phospholipids, and/or triglycerides in the blood serum of mammals.

The hyperlipidemias include six types of inheritable hyperlipoproteinemias; these types frequently are referred to as lipoprotein phenotypes. The major plasma lipids, including cholesterol and the triglycerides do not circulate freely in solution in plasma, but are bound to proteins and transported as macromolecular complexes called lipoproteins. Classification of inherited hyperlipoproteinemias according to phenotype is important, since dietary management and drug therapy are largely dependent on this information. (The Merck Manual, 16.sup.th edition, Robert Berkow and Andrew J. Fletcher, Merck & Co., Inc., Rahway, N.J. 1992). In the current practice of treating hyperlipidemia the goal is to lower lipid levels by weight control and diet control. As an adjunct to diet and weight control, blood lipid reducing agents, including, e.g., prescription drugs, may also be administered.

Plasma lipoproteins are carriers of lipids from the sites of synthesis and absorption to the sites of storage and/or utilization. Lipoproteins are spherical particles with triglycerides and cholesterol esters in their core and a layer of phospholipids, nonesterified cholesterol and apolipoproteins on the surface. Lipoproteins are categorized into five major classes based on their hydrated density as very large, triglyceride-rich particles known as chylomicrons, very low density lipoproteins (VLDL), intermediate-density lipoproteins (IDL), low-density lipoproteins (LDL) and, high-density lipoproteins (HDL).

Apolipoproteins are protein components of lipoproteins with three major functions which include: (1) maintaining the stability of lipoprotein particles, (2) acting as cofactors for enzymes that act on lipoproteins, and (3) removing lipoproteins from circulation by receptor-mediated mechanisms. The four groups of apolipoproteins are apolipoproteins A (Apo A), B (Apo B), C (Apo C) and E (Apo E).

LDL consists of a hydrophobic lipid core composed of cholesterol esters and triglycerides. The lipid core of the LDL particle is surrounded by an amphipathic coat composed of phospholipids, unesterified cholesterol and Apo B.

Several studies have shown that an increased Apo B level in blood is a reliable marker for coronary atherosclerosis (Sniderman, A. et al., Proc. Natl. Acad. Sci. USA, 77:604-608 (1980); Kwiterovich, P. O. et al., Am. J. Cardiol., 71:631-639 (1993); McGill et al. Coron. Artery Dis., 4:261-270 (1993); Tornvall, P. et al., Circulation, 88:2180-2189 (1993)).

In the United States, the complications of arteriosclerosis account for about one half of all deaths and for about one third of deaths in persons between 35 and 65 years of age. Atherosclerosis, or the development of atheromatous plaques in large and medium-sized arteries, is the most common form of arteriosclerosis. Many factors are associated with the acceleration of atherosclerosis, regardless of the underlying primary pathogenic change, for example, age, elevated plasma cholesterol level, high arterial blood pressure, cigarette smoking, reduced high-density lipoprotein (HDL) cholesterol levels, or family history of premature coronary artery disease.

The risk of death from coronary artery disease has a continuous and graded relation to total serum cholesterol levels greater than 180 mg/dl (Stamler, J. et al., (1986) JAMA 256:2823). Approximately one third of adults in the United States have levels that exceed 240 mg/dl and, therefore, have a risk of coronary artery disease that is twice that of people with cholesterol levels lower than 180 mg/dl. Acceleration of atherosclerosis is principally correlated with elevation of LDL, or beta fraction, has a negative correlation with atherosclerosis (Castelli, W. P. ct al. (1986) JAMA 256:2835). HDL exerts a protective effect and the ratio of total cholesterol to HDL cholesterol is a better predictor of coronary artery disease than the level of either alone. Total cholesterol levels are classified as being desirable (<200 mg/dl), borderline high (200-239 mg/dl), or high (>240 mg/dl)(Report of the National Education Program Expert Panel on Detection, Evaluation, and Treatment of High Blood Cholesterol in Adults (1988) Arch Intern Med 148:36).

Advances in the study of cholesterol metabolism and coronary disease have initiated an era of increased emphasis on preventive therapy. New guidelines for the detection and treatment of high blood cholesterol in adults recommend that patients with high cholesterol levels or with borderline-high levels and two or more additional risk factors should have a measurement of LDL. LDL cholesterol levels are then classified as borderline high risk (130-159 mg/dl) or high risk (>160 mg/dl). Dietary treatment is recommended for those patients with high-risk levels of LDL and for those with borderline-high risk levels who have two or more additional risk factors. Drug treatment is recommended for all patients with LDL levels greater than 189 mg/dl and for those patients with LDL cholesterol levels between 159 and 189 mg/dl who have two or more additional risk factors.

In view of the above, it is not surprising to find that a number of compounds have been proposed for the treatment of hyperlipidemia in mammals. For example, colestipol hydrochloride (U.S. Pat. Nos. 3,692,895 and 3,803,237) is a basic anion exchange resin which, when ingested, sequesters bile acids in the intestine. This stimulates the production of bile acids, which uses and depletes the body's stored cholesterol. This in turn reduces LDL levels. Gemfibrozil, described in U.S. Pat. No. 3,674,836 is also used in such treatment. Niacin (3-pyridinecarboxylic acid) is also administered for hypercholesterolemia, at a dosage of about 1.5 to 6 g/day orally. Other pharmaceutical agents occasionally administered for hyperlipidemia include neomycin, norethindrone acetate, oxandrolone, and dextrothyroxine (Remington's Pharmaceutical Sciences, (17th Ed., Mack Pub. Co., 1985), pp. 863-865). U.S. Pat. No. 4,499,303 describes the use of a class of N-benzoylsulfamates and benzoylsulfonamides as useful hypolipidemic agents. U.S. Pat. No. 4,395,417 proposes the use of cyclic imides, diones, reduced diones and analogs as useful agents.

The present invention relates to compositions and use thereof for the prevention and treatment of cardiovascular disease (e.g., hypercholesterolemia and atherosclerosis with combinations of flavanoids and tocotrienols. Flavonoids are polyphenolic compounds that occur unbiquitiously in plant foods especially in orange, grapefruit, and tangerine. Tocotrienols are present in palm oil and are a form of vitamin E having an unsaturated side chain. In the practice of the prevention and/or treatment of atherosclerosis and/or hypercholesterolemia, the flavonoids and tocotrienols are used to inhibit production of cholesterol, low-density lipoprotein (LDL) and Apo B protein. Compositions comprising citrus flavonoids and tocotrienols are used to prevent and/or inhibit production of total serum cholesterol, LDL and apoB.

### Flavonoids

Epidemiological studies have shown that flavonoids present in the Mediterranean diet may reduce the risk of death from coronary heart disease (Hertog, M. G. et al., 1993, Lancet: 342, 1007-1011). Soybean isoflavones for example, genistein, which is a minor component of soy protein preparations may have cholesterol-lowering effects (Kurowska, E. M. et al., 1990, J. Nutr. 120:831-836). The flavonoids present in citrus juices such as orange and grapefruit include, but are not limited to, hesperetin, and naringenin respectively. The flavonoids present in tangerine include, but are not limited to tangeretin or nobiletin.

Tocotrienols are present in palm oil and are a form of vitamin E having an unsaturated side chain. They include but are not limited to alpha-tocotrienol, gamma-tocotrienol or delta-tocotrienol.

In WO 02/34072 A2 and EP 1 415 549 A1 there are synergistic antioxidant combinations of tocols and polyphenols described. A source of α-tocotrienol and/or -tocopherol is described to act synergistically with an antioxidant source comprising polyphenols to effect suppression of LDL oxidation in serum. The combination of these two antioxidant sources has wide-ranging applications in treatment of medical conditions arising from free radical generation, including arteriosclerosis and cancer.

In WO 02/055071 A1 a pharmaceutical composition suitable for administering to a human subject at risk for or suffering from hypercholesterolemia is described, said composition comprising a cholesterol lowering effective amount of a limonoid selected from the group consisting of limonin and nomilin, and a flavonoid selected from the group consisting of hesperidin, naringin, naringenin, hesperitin, nobiletin and tangeretin. Also there is disclosed a pharmaceutical composition suitable for administering to a human subject at risk for or suffering from hypercholesterolemia, said composition comprising a cholesterol-lowering effective amount of a limonoid selected from the group consisting of limonin and nomilin, and a tocotrienol. In table 6 on page 28 there are several combinations of tocotrienols and flavonoids showing a reduction in the HDL level accompanying the overall reduction in cholesterol levels.

There exists a further need in the art for compounds comprising flavonoids and tocotrienols for preventing and treating cardiovascular disease.

### SUMMARY OF THE INVENTION

It is an object of the present invention to provide a pharmaceutical ingredient and/or formulation for treating and/or preventing cardiovascular disease comprising flavonoids and tocotrienols.

It is a further object of the present invention to provide compositions for use in treating cardiovascular diseases by administering a pharmaceutical ingredient and/or formulation comprising flavonoids and tocotrienols.

It is another object of the invention is to provide ingredients/formulations to treat atherosclerosis or hypercholesterolemia by lowering total cholesterol utilizing flavonoids and tocotrienols.

It is another object of the invention is to provide ingredients/formulations to treat atherosclerosis or hypercholesterolemia by lowering triacylglycerols utilizing flavonoids and tocotrienols.

It is another object of the invention is to provide ingredients/formulations to treat atherosclerosis or hypercholesterolemia by lowering LDL cholesterol utilizing flavonoids and tocotrienols.

It is another object of the invention is to provide ingredients/formulations to treat atherosclerosis or hypercholesterolemia by lowering Apo B utilizing flavonoids and tocotrienols.

It is another object of the invention is to provide ingredients/formulations to treat atherosclerosis or hypercholesterolemia by utilizing flavonoids and tocotrienols, wherein the ingredients/formulations have low levels of synephrine.

Certain of the above objects of the invention can be achieved by the present invention which in certain embodiments is directed to a pharmaceutical ingredient comprising an active agent combination comprising polymethoxylated flavonoids and tocotrienols in a ratio of about 90:10_to about 95:5, the pharmaceutical ingredient selected from the group consisting of an essence oil isolated from a citrus fruit, a peel oil isolated from a citrus fruit, a peel isolated from a citrus fruit, decharacterized citrus fruit, and combinations thereof.

In certain embodiments, the invention is directed to a pharmaceutical formulation comprising a pharmaceutical ingredient comprising an active agent combination comprising flavonoids and tocotrienols in a ratio of 90:10 to about 95:5, the pharmaceutical ingredient selected from the group consisting of an essence oil isolated from a citrus fruit, a peel oil isolated from a citrus fruit, a peel isolated from a citrus fruit, decharacterized citrus fruit, and combinations thereof and at least one pharmaceutically acceptable excipient.

In certain embodiments, the invention is directed to a pharmaceutical formulation comprising a pharmaceutical ingredient comprising an active agent combination comprising 90:10 to about 95:5, the pharmaceutical ingredient selected from the group consisting of an essence oil isolated from a citrus fruit, a peel oil isolated from a citrus fruit, a peel isolated from a citrus fruit, decharacterized citrus fruit, and combinations thereof and at least one pharmaceutically acceptable excipient, the formulation lowering total cholesterol by at least 10% after administration.

In certain embodiments, the invention is directed to a pharmaceutical formulation comprising a pharmaceutical ingredient comprising an active agent combination comprising 90:10 to about 95:5, the pharmaceutical ingredient selected from the group consisting of an essence oil isolated from a citrus fruit, a peel oil isolated from a citrus fruit, a peel isolated from a citrus fruit, decharacterized citrus fruit, and combinations thereof and at least one pharmaceutically acceptable excipient, the formulation lowering triaglycerols by at least 15% after administration.

In certain embodiments, the invention is directed to a pharmaceutical formulation comprising a pharmaceutical ingredient comprising an active agent combination comprising 90:10 to about 95:5, the pharmaceutical ingredient selected from the group consisting of an essence oil isolated from a citrus fruit, a peel oil isolated from a citrus fruit, a peel isolated from a citrus fruit, decharacterized citrus fruit, and combinations thereof and at least one pharmaceutically acceptable excipient, the formulation lowering LDL cholesterol by at least 10% after administration.

In certain embodiments, the invention is directed to a pharmaceutical formulation comprising a pharmaceutical ingredient comprising an active agent combination comprising 90:10 to about 95:5, the pharmaceutical ingredient selected from the group consisting of an essence oil isolated from a citrus fruit, a peel oil isolated from a citrus fruit, a peel isolated from a citrus fruit, decharacterized citrus fruit, and combinations thereof and at least one pharmaceutically acceptable excipient, the formulation lowering Apo B by at least 10% after administration.

The term "essence oil" refers to the oil-soluble components (e.g., fraction) remaining after evaporation of a fruit juice.

The term "peel oil" refers to oil isolated from the peel of a citrus fruit.

The term "peel" refers to the peel of a citrus fruit which, for purposes of the present invention, may be e.g., dried, shredded, or pelletized.

The term "citrus fruit" refers to a fruit from the genus Citrus that includes, e.g., orange, lemon, lime, tangerine, grapefruit (e.g., pink grapefruit, red peel grapefruit) and, in particular, citrus arurentium.

The term "decharacterized fruit" refers to fruit from which the juice has been extracted. The decharacterized fruit can be in the form of, for example, a mash or presscake. The term "Tomah presscake" refers to a particularly preferred presscake described in U.S. Pat. Nos. 5,320,861 and 5,320,861 which contains higher levels of desirable phytochemicals than are present in presscake made via conventional methods. In particular, decharacterized cranberry fruit in the form of "Tomah presscake" contains higher levels of anthocyanins, phenolic acids and proanthocyanidins than that found in presscake produced through conventional methods. For example, the anthocyanin content is typically 30% or greater of that present in native cranberry fruit, the phenolic acid content is typically 8% or greater of that present in native cranberry fruit and the proanthocyanidin content is typically 60% or greater of that present in native cranberry fruit.

The term "isolated" refers to the removal or change of a composition or compound from its natural context.

The term "flavonoid" includes, but is not limited to polymethoxylated flavonoids and refers to any member of the group of aromatic, oxygen-containing, heterocyclic pigments found in the derivatives of the invention and includes for example members of the chemical subgroups 1) catechins, 2) leucoanthocyanidins and flavanones, 3) flavanins, flavones, and anthocyanins, and 4) flavonols. In preferred embodiments, a flavonoid includes, e.g., a proanthocyanidin, flavan-3-ol, anthocyanin, or flavanol. The flavonoid can include e.g., naringenin, hesperetin, nobiletin, and/or tangeretin

The term "pharmaceutical ingredient" means a therapeutic composition which can be optionally combined with pharmaceutically acceptable excipients to provide a pharmaceutical formulation or dosage form.

The term "pharmaceutical formulation" means a pharmaceutical ingredient in combination with at least one pharmaceutically acceptable excipient. The formulation can be administered by any acceptable route, e.g., oral in any acceptable form, e.g., a tablet or capsule.

### DETAILED DESCRIPTION OF THE INVENTION

In certain embodiments, the present invention is directed to a pharmaceutical ingredient comprising an active agent combination comprising polymethoxylated flavonoids and tocotrienols in a ratio of about 90:10 to about 95:5, the pharmaceutical ingredient selected from the group consisting of an essence oil isolated from a citrus fruit, a peel oil isolated from a citrus fruit, a peel isolated from a citrus fruit, decharacterized citrus fruit, and combinations thereof.

In certain embodiments, the active agent combination comprises flavonoids and tocotrienols in a ratio of about 90:10; or in a ratio of about 95:5.

In certain embodiments, the pharmaceutical ingredient of the present invention comprising from about 50% to about 90% of flavonoids and tocotrienols; from about 60% to about 80% of the active agent combination; or about 70% of the active agent combination.

In certain embodiments, the pharmaceutical ingredient contains less than about 1% synephrine; less than about 0.5% synephrine; or less than 0.1% synephrine.

In certain embodiments, the formulation of the contains less than about 1% synephrine; less than about 0.5% synephrine; or less than 0.1% synephrine.

The flavonoid of the present invention is a polymethoxylated flavonoid. In certain embodiments, the flavonoid comprises a member selected from the group considting of naringenin, hesperetin, nobiletin, tangeretin and combinations thereof.

The tocotrienol of the present invention can be, e.g., selected from the group consisting of alpha-tocotrienol, gamma-tocotrienol, delta-tocotrienol, and combinations thereof.

In certain embodiments, the invention is directed to a pharmaceutical formulation comprising a pharmaceutical ingredient comprising an active agent combination comprising polymethoxylated flavonoids and tocotrienols in a ratio of about 90:10 to about 95:5, the pharmaceutical ingredient selected from the group consisting of an essence oil isolated from a citrus fruit, a peel oil isolated from a citrus fruit, a peel isolated from a citrus fruit, decharacterized citrus fruit, and combinations thereof and at least one pharmaceutically acceptable excipient.

In certain embodiments, the pharmaceutical ingredient of the formulation of the present invention comprises is in an effective amount to treat a human subject at risk of or suffering from a cardiovascular disease, e.g., hypercholesterolemia or atherosclerosis.

In certain embodiments, the pharmaceutical formulation of the present invention is suitable for administration intravenously, intraperitoneally, subcutaneously, intramuscularly, intrathecally, orally, rectally, topically, or by inhalation.

In certain embodiments, the pharmaceutical formulation of the present invention is in the form of a tablet, a capsule, a solution, a liquid, a suspension, or an emulsion.

In certain embodiments, the pharmaceutical formulation of the present invention comprises from about 60 mg of the tocotrienol and about 560 mg of the flavonoid per unit dose; from about 10 mg to about 80 mg of the tocotrienol and from about 150 mg to about 750 mg of the flavonoid per unit dose; or about 30 mg of the tocotrienol and about 270 mg of the flavonoid per unit dose.

In the methods of the present invention, the daily dose of the active agents can be, e.g., from about 60 mg of the tocotrienol and about 560 mg of the flavonoid; from about 10 mg to about 80 mg of the tocotrienol and from about 150 mg to about 750 mg of the flavonoid; or about 30 mg of the tocotrienol and about 270 mg of the flavonoid.

In the methods of the present invention, the flavonoids and the tocotrienols can be administered in the same dosage form or in separate dosage forms. Further, the flavonoids and tocotrienols can be administered by the same route of administration or by different routes of administration.

In certain embodiments, the pharmaceutical formulation of the present invention lowers total cholesterol by at least 10%, 20% or 30%.

In certain embodiments, the pharmaceutical formulation of the present invention lowers total cholesterol by at least 10%, 20% or 30% in a single patient or in a patient population; after single dose administration, multiple dose administration (e.g., after four weeks) or after steady state administration.

In certain embodiments, the pharmaceutical formulation of the present invention lowers triacylglycerols by at least 15%, 25% or 35%.

In certain embodiments, the pharmaceutical formulation of the present invention lowers triacylglycerols by at least 15%, 25% or 35% in a single patient or in a patient population; after single dose administration, multiple dose administration (e.g., after four weeks) or after steady state administration.

In certain embodiments, the pharmaceutical formulation of the present invention lowers LDL cholesterol by at least 10%, 20% or 30%.

In certain embodiments, the pharmaceutical formulation of the present invention lowers LDL cholesterol by at least 10%, 20% or 30% in a single patient or in a patient population; after single dose administration, multiple dose administration (e.g., after four weeks) or after steady state administration.

In certain embodiments, the pharmaceutical formulation of the present invention lowers Apo B by at least 10%, 20% or 30%.

In certain embodiments, the pharmaceutical formulation of the present invention lowers total cholesterol by at least 10%, 20% or 30% in a single patient or in a patient population; after single dose administration, multiple dose administration (e.g., after four weeks) or after steady state administration.

In certain embodiments, the pharmaceutical formulation of the present invention comprises an active agent combination of the present invention and an additional active agent selected from the group consisting of soy protein, soy isoflavones, grapeseed extract, pine bark extract, gugulipids, policosinols, pantesine, niacin, alpha lipoic acid, tea flavins, coenzyme q10, lutein, statins, and combimations thereof.

In certain embodiments, the statin drug is selected from the group consisting of pravastatin, simvastatin, lovastatin, atorvastatin, fluvastatin, cerivastatin and combinations thereof.

The pharmaceutical formulations of the present invention can be prepared as oral, sublingual, inhaled, subcutaneous, intramuscular, intravenous, transdermal, and formulations for local or rectal administration. Oral formulations can be in the form of, e.g., tablets, gel capsules, powders, granules and oral solutions or suspensions, sublingual and buccal administration forms.

When a solid composition is prepared in the form of tablets or gel capsules, a mixture of pharmaceutical excipients which can be composed of diluents such as, for example, lactose, microcrystalline cellulose, starch, dicalcium phosphate, binders such as, for example, polyvinylpyrrolidone, hydroxypropylmethylcellulose, crumbling agents such as crosslinked polyvinylpyrrolidone, crosslinked carboxymethyl-cellulose, flow agents such as silica or talc, and lubricants such as magnesium stearate, stearic acid, glyceryl tribehenate or sodium stearyl fumarate, is added to the micronized or non-micronized active principle.

Wetting agents or surfactants such as sodium lauryl sulphate, polysorbate 80 or poloxamer 188 can be added to the formulation.

The tablets can be prepared by various techniques: direct tabletting, dry granulation, wet granulation, hot-melt.

The tablets may be uncoated coated (e.g., with sucrose) or coated with various polymers (e.g., hydroxypropylmethylcellulose) or other suitable materials.

The tablets can have immediate, delayed or sustained release by preparing matrices or by using coatings.

The gel capsules can be soft or hard, and coated with film or otherwise, so as to have immediate, sustained or delayed activity (for example via an enteric form).

Oral formulations can also be prepared as liquid or semi-solid formulations, as e.g., a preparation in the form of a syrup or elixir can contain the active principle together with a sweetener, preferably a calorie-free sweetener, methyl paraben and propyl paraben as antiseptic agent, as well as a flavouring agent and a suitable colorant.

Water-dispersible powders or granules can contain the active principle as a mixture with dispersants, wetting agents or suspending agents, such as polyvinylpyrrolidone, as well as with sweeteners or flavour enhancers.

For rectal administration, use is made of suppositories which are prepared with binders that melt at the rectal temperature, for example cocoa butter or polyethylene glycols.

Aqueous suspensions, isotonic saline solutions or sterile, injectable solutions which contain pharmacologically compatible dispersants and/or solubilizing agents, for example propylene glycol, are used for parenteral or intranasal administration.

Thus, in order to prepare an aqueous solution which can be injected intravenously, a co-solvent such as, for example, an alcohol such as ethanol or a glycol such as polyethylene glycol or propylene glycol, and a hydrophilic surfactant such as polysorbate 80 or poloxamer 188 can be used. To prepare an injectable oily solution for intramuscular administration, the active principle can be dissolved with a triglyceride or a glycerol ester.

Creams, ointments, gels, transdermal patches and sprays can be used for local administration. Patches in multilaminar or reservoir form in which the active principle can be in alcoholic solution, and sprays can be used for transdermal administration.

An aerosol containing, for example, sorbitan trioleate or oleic acid as well as trichlorofluoromethane, dichlorofluoromethane, dichlorotetrafluoroethane, freon substitutes or any other biologically compatible propellent gas is used for administration by inhalation; a system containing the active principle alone or combined with an excipient, in powder form, can also be used.

The active principle can also be formulated in the form of microcapsules or microspheres, optionally with one or more supports or additives.

Among the sustained-release forms which are useful in the case of chronic treatments, it is possible to use implants. These can be prepared in the form of an oily suspension or in the form of a suspension of microspheres in an isotonic medium.

### EXAMPLE 1

The effect of a combination of flavones and tocotrienols on lowering cholesterol in human subjects were studied. Study Design:

Ten hypercholesterolemic subjects with serum total cholesterol >5.9 mmol/L, LDL cholesterol 4.0 mmol/L and serum triacylglycerols <3.5 mmol/L (>230 mg/dl, >155 mg/dl and <307 mg/dl, respectively) were given a daily supplement consisting of 270 mg polymethoxyflavones and 30 mg tocotrienols for four weeks. To be included in this study, the subjects had to be free of thyroid disorders, kidney disorders and diabetes. Also, subjects taking cholesterol-lowering medications were asked to discontinue the treatment four weeks before the study.

To determine whether this treatment improved parameters associated with high risk of heart disease fasting blood samples were drawn at the onset of the study and at the end of a 4-week for analysis of plasma total and lipoprotein cholesterol, plasma apolipoprotiens B (associated with LDL) and Al (associated with HDL), total triacylglycerols. The protocol was approved by the Human Ethics Committee of the University of Western Ontario and informed consent was obtained from each subject.

Cholesterol and triacylglycerols were measured with enzymatic timed-endpoint methods by using CHOL Reagent or Triacylglycerol GPO reagent. Plasma concentrations of apo B and apo A1 were analyzed immunoephelometrically with a BNII System.

Subjects were instructed to maintain their caloric intake during the study. This was measured by measurement of Body Mass Index (BMI) before and after treatment.

Changes from baseline after four weeks were analyzed by using repeated-measures analysis of variance (ANOVA) followed by Dunnet's t tests. Results:

Treatment with the combination of 90% flavonoid and 10% tocotrienol was associated with a number of beneficial effects. Treatment associated with this combination was associated with a significant reduction in total cholesterol, LDL cholesterol and serum triacylglycerols. See Table 1. The results suggest that the combination of flavonoid and tocotrienol lowers cholesterol in humans.

**Table 1**

| Variable | Baseline | 4 weeks |
|---|---|---|
| Body mass index (kg/m2) | 28.8 ∓ 4.6 | 28. 28.6 ∓ 4.5 |
| Total cholesterol (mg/dl) | 266.82 ∓ 34.80 | 01.08 ∓ 27.07 |
| VLDL cholesterol (mg/dl) | 30.94 ∓ 11.6 | 34.80 ∓ 15.47 |
| LDL cholesterol (mg/dl) | 181.75 ∓ 30.94 | 146.95 ∓ 27.07 |
| HDL cholesterol (mg/dl) | 43.54 ∓ 11.60 | 42.54 ∓ 11.60 |

### EXAMPLE 2

Clinical Trial 2: Cholesterol-Lowering Properties of Combination of Polymethoxylated Flavones and Tocotrienols in Human Subjects.

The objective of this study was to evaluate the cardio protective potential of a combination of flavones and tocotrienols in human subjects. Study Design:

Ten hypercholesterolemic subjects were given a daily supplement consisting of 270 mg polymethoxyflavones and 30 mg tocotrienols for four weeks. To be included in this study, the subjects had to be free of thyroid disorders, kidney disorders and diabetes. Also, subjects taking cholesterol-lowering medications were asked to discontinue the treatment four weeks before the study.

Blood samples were taken from the forearm vein before the start (baseline), and at the end of the 4 week period. Plasma lipids profiles and other metabolic parameters were analyzed using standard methods. Blood pressure was recorded in the sitting position, using a conventional mercury manometer, by calculating a mean of two readings. Results:

Treatment with the combination of 90% flavonoid and 10% tocotrienol was associated with a number of beneficial effects. Treatment associated with this combination was associated with a reduction in total cholesterol (19.7%), LDL cholesterol (22,01%), serum triacylglycerols (28.4%), apo B (20.9%), and systolic blood pressure. See Table 2. The results suggest that the combination of flavonoid and tocotrienol has cardio protective potential in subjects with moderate hypercholesterolemia.

**Table 2**

| Variable | Week 0 | Week 4 |
|---|---|---|
| Systolic blood pressure, mmHg | 123.0 ∓ 22.4 | 116.0 ∓ 12.7 |
| Diastolic blood pressure, mmHg | 79.0 ∓ 13.0 | 76.0 ∓ 9.2 |
| Body weight, kg | 80.8 ∓ 10.9 | 81.1 ∓ 11.7 |
| Body Mass Index (BMI), kg/m2 | 27.4 ∓ 1.8 | 27.5 ∓ 1.9 |
| Total cholesterol, mmol/L | 255.61 ∓ 35.96 | 205.34 ∓ 18.95 |
| Triacylglycerols, mmol/L | 77.34 ∓ 29.78 | 55.41 ∓ 13.64 |
| HDL cholesterol, mmol/L | 42.92 ∓ 9.67 | 44.08 ∓ 8.51 |
| LDL cholesterol, mmol/L | 197.60 ∓ 34.03 | 154.29 ∓ 16.63 |

## Claims

1. A pharmaceutical ingredient comprising an active agent combination comprising polymethoxylated flavonoids and tocotrienols in a ratio of about 90:10 to about 95:5, the pharmaceutical ingredient selected from the group consisting of an essence oil isolated from a citrus fruit, a peel oil isolated from a citrus fruit, a peel isolated from a citrus fruit, de**characterized** citrus fruit, and combinations thereof.

2. The pharmaceutical ingredient according to claim 1, wherein the active agent combination comprises flavonoids and tocotrienols in a ratio of about 90:10.

3. The pharmaceutical ingredient according to claim 1, wherein the active agent combination comprises flavonoids and tocotrienols in a ratio of about 95:5.

4. The pharmaceutical ingredient according to claim 1, comprising from about 50% to about 90% of the active agent combination.

5. The pharmaceutical ingredient according to claim 1, comprising from about 60% to about 80% of the active agent combination.

6. The pharmaceutical ingredient according to claim 1, comprising about 70% of the active agent combination.

7. The pharmaceutical ingredient according to claim 1, wherein the flavonoid comprises a member consisting of nobiletin and tangeretin.

8. The pharmaceutical ingredient according to claim 1, wherein the tocotrienol is selected from the group consisting of alpha-tocotrienol, gamma-tocotrienol, and delta-tocotrienol.

9. A pharmaceutical formulation comprising a pharmaceutical ingredient according to claim 1 and at least one pharmaceutically acceptable excipient.

10. The pharmaceutical formulation according to claim 9 for the use of lowering total cholesterol by at least 10%.

11. The pharmaceutical formulation according to claim 9 for the use of lowering triacylglycerols by at least 15%.

12. The pharmaceutical formulation according to claim 9 for the use of lowering LDL cholesterol by at least 10%.

13. The pharmaceutical formulation according to claim 9 for the use of lowering Apo B by at least 10%.

14. A pharmaceutical formulation comprising a pharmaceutical ingredient according to claim 1 and an additional active agent selected from the group consisting of soy protein, soy isoflavones, grapeseed extract, pine bark extract, gugulipids, policosinols, pantesine, niacin, alpha lipoic acid, tea flavins, coenzyme q10, lutein, statins, and combinations thereof.

15. A pharmaceutical formulation according to claim 9 for the use in the treatment of a human subject at risk of or suffering from cardiovascular disease.

16. The pharmaceutical formulation for use of claim 15, wherein the cardiovascular disease is hypercholesterolemia or atherosclerosis.

17. The pharmaceutical formulation for use of claim 16, wherein the active agent combination comprises flavonoids and tocotrienols in a ratio of about 95:5.

## Patentansprüche

1. Arzneimittelwirkstoff umfassend eine Wirkstoffkombination, wobei die Wirkstoffkombination polymethoxylierte Flavonoide und Tocotrienole in einem Verhältnis von ungefähr 90:10 bis zu einem Verhältnis von ungefähr 95:5 umfasst, wobei der Arzneimittelwirkstoff ausgewählt ist aus der Gruppe bestehend aus einem aus einer Zitrusfrucht isolierten Essenzöl, ein aus einer Zitrusfrucht isoliertes Schalenöl, eine von einer Zitrusfrucht isolierte Schale, eine decharakterisierte Zitrusfrucht und Kombinationen davon.

2. Arzneimittelwirkstoffnach Anspruch 1, wobei die Wirkstoffkombination Flavonoide und Tocotrienole in einem Verhältnis von ungefähr 90:10 umfasst.

3. Arzneimittetwirkstoff nach Anspruch 1, wobei die Wirkstoffkombination Flavonoide und Tocotrienole in einem Verhältnis von ungefähr 95:5 umfasst.

4. Arzneimittelwirkstoff nach Anspruch 1, wobei der Arzneimittelwirkstoff ungefähr 50% bis ungefähr 90% von der Wirkstoffkombination umfasst.

5. Arzneimittelwirkstoff nach Anspruch 1, wobei der Arzneimittelwirkstoff ungefähr 60% bis ungefähr 80% von der Wirkstoffkombination umfasst.

6. Arzneimittelwirkstoff nach Anspruch 1, wobei der Arzneimittelwirkstoff ungefähr 70% von der Wirkstoffkombination umfasst.

7. Arzneimittelwirkstoff nach Anspruch 1, wobei das Flavonoid ein Mitglied bestehend aus Nobiletin und Tangeretin umfasst.

8. Arzneimittelwirkstoff nach Anspruch 1, wobei das Tocotrienol ausgewählt ist aus der Gruppe bestehend aus alpha-Tocotrienol, gamma-Tocotrienol und delta-Tocotrienol.

9. Arzneimittel-Formulierung umfassend einen Arzneimittelwirkstoff nach Anspruch 1 und mindestens einen pharmazeutisch annehmbaren Exzipienten.

10. Arzneimittel-Formulierung nach Anspruch 9 für die Verwendung zum Erniedrigen des gesamten Cholesterins um mindestens 10%.

11. Arzneimittel-Formulierung nach Anspruch 9 für die Verwendung zum Erniedrigen des Triacylglycerols um mindestens 15%.

12. Arzneimittel-Formulierung nach Anspruch 9 für die Verwendung zum Erniedrigen des LDL-Cholesterins um mindestens 10%.

13. Arzneimittel-Formulierung nach Anspruch 9 für die Verwendung zum Erniedrigen von ApoB um mindestens 10%.

14. Arzneimittel-Formulierung umfassend einen Arzneimittelwirkstoff nach Anspruch 1 und einen weiteren Wirkstoff ausgewählt aus der Gruppe bestehend aus Sojaprotein, Sojaisoflavone, Traubenkemextrakt, Kiefernrindeextrakt, Gugulipide, Policosinol, Pantesin, Niacin, alpha- Liponsäure, Teeflavine, Coenzym q10, Lutein, Statine und Kombinationen davon.

15. Arzneimittel-Formulierung nach Anspruch 9 zur Verwendung bei der Behandlung eines menschlichen Patienten, bei dem die Gefahr besteht an einer kardiovaskulären Krankheit zu leiden oder der bereits an einer kardiovaskulären Krankheit leidet.

16. Arzneimittel-Formulierung zur Verwendung nach Anspruch 15, wobei die kardiovaskulären Krankheit Hypercholesterinämie oder Atherosklerose ist.

17. Arzneimittel-Formulierung zur Verwendung nach Anspruch 16, wobei die Wirkstoffkombination Flavonoide und Tocotrienole in einem Verhältnis von ungefähr 95:5 umfasst.

## Revendications

1. Ingrédient pharmaceutique comprenant une combinaison d'agent actif comprenant des flavonoïdes polyméthoxylés et des tocotriénols dans un rapport d'environ 90:10 à environ 95:5, l'ingrédient pharmaceutique étant sélectionné à partir du groupe constitué par une huile d'essence isolée à partir d'un agrume, une huile de pelure isolée à partir d'un agrume, une pelure isolée à partir d'un agrume, un agrume dé**caractérisé** et des combinaisons de ceux-ci.

2. Ingrédient pharmaceutique selon la revendication 1, dans lequel la combinaison d'agent actif comprend des flavonoïdes et des tocotriénols dans un rapport d'environ 90:10.

3. Ingrédient pharmaceutique selon la revendication 1, dans lequel la combinaison d'agent actif comprend des flavonoides et des tocotriénols dans un rapport d'environ 95:5.

4. Ingrédient pharmaceutique selon la revendication 1, comprenant environ 50 % à environ 90 % de la combinaison d'agent actif.

5. Ingrédient pharmaceutique selon la revendication 1, comprenant environ 60 % à environ 80 % de la combinaison d'agent actif.

6. Ingrédient pharmaceutique selon la revendication 1, comprenant environ 70 % de la combinaison d'agent actif.

7. Ingrédient pharmaceutique selon la revendication 1, dans lequel le flavonoïde comprend un élément consistant en nobilézine et en tangeretine.

8. Ingrédient pharmaceutique selon la revendication 1, dans lequel le tocotriénol est sélectionné dans le groupe constitué par l'alpha-tocotriénol, le gamma-tocotriénol et le delta-tocotriénol.

9. Formulation pharmaceutique comprenant un ingrédient pharmaceutique selon la revendication 1, et au moins un excipient acceptable d'un point de vue pharmaceutique.

10. Formulation pharmaceutique selon la revendication 9, à utiliser pour l'abaissement du cholestérol total d'au moins 10 %.

11. Formulation pharmaceutique selon la revendication 9, à utiliser pour l'abaissement des triglycérides d'au moins 15 %.

12. Formulation pharmaceutique selon la revendication 9, pour l'utilisation pour l'abaissement du cholestérol LDL d'au moins 10 %.

13. Formulation pharmaceutique selon la revendication 9, pour l'utilisation pour l'abaissement de l'Apo B d'au moins 10 %.

14. Formulation pharmaceutique comprenant un ingrédient pharmaceutique selon la revendication 1 et un agent actif additionnel sélectionné à partir du groupe constitué par une protéine de soja, des isoflavones de soja, un extrait de pépin de raisin, un extrait d'écorce de pin, des gugulipides, des policosinols, de la pantésine, de l'acide nicotinique, de l'acide alpha-lipoïque, des flavines de thé, une coenzyme q10, de la lutéine, des statines, et des combinaisons de ceux-ci.

15. Formulation pharmaceutique selon la revendication 9, pour l'utilisation dans le traitement d'un sujet humain risquant ou souffrant d'une maladie cardiovasculaire.

16. Formulation pharmaceutique ou utilisation selon la revendication 15, dans laquelle la maladie cardiovasculaire est l'hypercholestérolémie ou l'athérosclérose.

17. Formulation pharmaceutique ou utilisation selon la revendication 16, dans laquelle la combinaison d'agent actif comprend des flavonoïdes et des tocotriénols dans un rapport d'environ 95:5.
